# EUROPEAN PATENT APPLICATION

(11) **EP 1 813 670 A1**
(43) Date of publication of application: **01.08.2007**
(21) Application number: 05788124.5
(22) Date of filing: 03.10.2005
(51) Int. Cl.: C12N 15/00, C12N 15/09, C12N 1/19

(54) **PROCESS FOR PRODUCING COHESIVE ALCOHOL FERMENTATION YEAST AND COHESIVE ALCOHOL FERMENTATION YEAST**

(30) Priority: 08.10.2004 JP 2004296638
(71) Applicant: Sapporo Breweries Limited, Tokyo 150-8522 (JP)
(72) Inventor: FUJII, Keiko, Tokyo 130-0021 (JP); GOTO, Shingo, Oyumino Heim C-312, Chiba-shi, Chiba 2660033 (JP)
(74) Representative: Vossius & Partner
(86) International application number: PCT/JP2005/018286
(87) International publication number: WO 2006/040955

(57) **Abstract**

The invention provides a method for production of non-uracil-requiring flocculant alcohol-fermenting yeast, comprising a step of introducing the *Saccharomyces cerevisiae-*derived URA3 gene only at one of the two LEU2 loci on the chromosomes of *Saccharomyces cerevisiae* strain FSC27 (FERM P-16023).

## Description

### Technical Field

The present invention relates to a method for production of flocculant alcohol-fermenting yeast, and to flocculant alcohol-fermenting yeast.

### Background Art

Alcohol production by fermentation methods is carried out using particularly high alcohol-producing strains (hereinafter referred to as "alcohol-fermenting yeast") of the fermenting yeast *Saccharomyces cerevisiae*, based on batch fermentation processes. Repeated batch fermentation using flocculant yeast is one such method which can improve operation efficiency and production efficiency for alcohol production.

Flocculant yeast are yeast of a nature such that the individual cells interact asexually to form flocculates which settle to liquid bottoms in stationary medium (hereinafter referred to as "flocculant"). Flocculant yeast obtained from natural sources usually have insufficient alcohol production ability, and are poorly suited for practical use as yeast for industrial alcohol production. The present inventors have created a self-cloning strain FSC27 (National Institute of Bioscience and Human Technology; currently, National Institute of Advanced Industrial Science and Technology, International Patent Organism Depositary; Tsukuba Central 6, 1-1-1 Higashi, Tsukuba, Ibaraki, Japan, 305-8566; Accession Number: FERM P-12804), obtained by introducing the flocculant gene FLO1 into the non-flocculant alcohol-fermenting yeast strain 396-9-6V (Deposited on 7th January 1997; Accession Number: FERM P-16023) (Patent document 1).

[Patent document 1]
   Japanese Patent No. 3040959

### Disclosure of the Invention

### Problems to be Solved by the Invention

The produced ethanol concentration of strain FSC27 is equivalent to that of the parental strain 396-9-6V, but the fermentation speed is slower than 396-9-6V. This problem results because strain FCS27 having FLO 1 introduced at the URA3 locus is a uracil-requiring strain. Culturing of FSC27 in medium containing an appropriate amount of added uracil produces a fermentation speed equivalent to strain 396-9-6V, but this method is not practical.

It is therefore an object of the present invention to provide a flocculant alcohol-fermenting yeast which exhibits a sufficient ethanol production ability and fermentation speed even in uracil-free medium.

### Means for Solving the Problems

In order to achieve the object stated above, the invention provides a method for production of non-uracil-requiring flocculant alcohol-fermenting yeast, comprising a step of introducing the *Saccharomyces cerevisiae-derived* URA3 gene only at one of the two LEU2 loci on the chromosomes of *Saccharomyces cerevisiae* strain FSC27. Introduction of the URA3 gene at a LEU2 locus allows transformation of the yeast into a uracil-non-requiring strain while maintaining high alcohol production ability.

The invention further provides non-uracil-requiring flocculant alcohol-fermenting yeast, having the *Saccharomyces cerevisiae*-derived URA3 gene introduced only at one of the two LEU2 loci on the chromosomes of *Saccharomyces cerevisiae* strain FSC27. The flocculant alcohol-fermenting yeast is preferably *Saccharomyces cerevisiae* strain FSCU-L18 (National Institute of Advanced Industrial Science and Technology, International Patent Organism Depositary; Tsukuba Central 6, 1-1-1 Higashi, Tsukuba, Ibaraki, Japan, 305-8566; Deposited on 20th May 2004; Accession Number: FERM P-20055). Yeast having the URA3 gene introduced at a LEU2 locus exhibit both high alcohol producing ability and lack of uracil requirement. Strain FSCU-L18, in particular, exhibits high alcohol producing ability and is therefore suitable as yeast for industrial alcohol production.

### Effect of the Invention

According to the invention it is possible to provide a non-uracil-requiring flocculant alcohol yeast having an alcohol production ability and fermentation speed which is superior to strain FSC27.

### Brief Description of the Drawings

Fig. 1 is a schematic illustration of double fusion PCR.
Fig. 2 is a drawing illustrating a strategy for introducing URA3 into a LEU2 locus.
Fig. 3 shows the results of Southern blotting of strain 396-9-6V. Lane 1: YRpGL10/BamHI, Lane 2: EcoRV, Lane 3: EcoRI, Lane 4: indIII, Lane 5: BamHI, Lane 6: PstI.
Fig. 4 shows the results of Southern blotting of strain FSCU-L. (a) shows the results using URA3 ORF as a probe, (b) shows the results using LEU2 ORF as a probe and (c) shows the results using pBR322/HindIII as a probe. Lane M: YRpGL10, Lane 1: strain FSC27, Lane 2: strain FSCU-L18, Lane 3: strain FSCU-L20, Lane 4: strain FSCU-L21.
Fig. 5 is a graph showing carbon dioxide generation in strain FSCU-L flask fermentation test.
Fig. 6 is a graph showing carbon dioxide generation in repeated batch fermentation using strain FSCU-L 18.
Fig. 7 is a graph showing carbon dioxide generation in repeated batch fermentation using strain FSC27.
Fig. 8 is a graph showing time required for fermentation with repeated batch fermentation using strain FSCU-L18 and strain FSC27.

### Best Modes for Carrying Out the Invention

The present invention will now be explained in greater detail.

First, a method for production of non-uracil-requiring flocculant alcohol-fermenting yeast of the invention will be explained. The method for production of non-uracil-requiring flocculant alcohol-fermenting yeast of the invention comprises a step of introducing the *Saccharomyces cerevisiae-derived* URA3 gene at only one of the two LEU2 loci on the chromosomes of *Saccharomyces cerevisiae* strain FSC27.

Strain FSC27, having the flocculant yeast FLO1 gene introduced and the URA3 gene disrupted, is uracil-requiring. Thus, introduction of the *Saccharomyces cerevisiae-derived* URA3 gene at only one of the two LEU2 loci on the strain FSC27 chromosomes produces a uracil-non-requiring transformant of strain FSC27. Since the introduced URA3 gene is derived from *Saccharomyces cerevisiae,* its recombination is self-cloning. Thus, it falls outside the scope of regulations such as guidelines for recombinant DNA experimentation, and is suitable for practical use, including utilization of transformants obtained in alcohol production utilizing existing equipment.

The *Saccharomyces cerevisiae-derived* URA3 gene used may be one isolated and prepared from chromosomes, or already isolated on a plasmid. An example of such a plasmid which may be used is YIp5. Also, double fusion PCR may be employed to prepare a LEU2-dismpting DNA fragment containing the full-length URA3 gene (see the examples for details regarding double fusion PCR). The URA3 gene is then introduced into strain FSC27 by a publicly known technique such as the lithium acetate method.

The obtained transformants are cultured in uracil-free medium and transformants are selected, to obtain only transformants having the URA3 gene introduced at only one LEU2 locus.

The transformants obtained in this manner, having the URA3 gene introduced at only one LEU2 locus, are used in the alcohol fermentation test described in the Examples, allowing strains with particularly superior alcohol producing ability to be selected. Confirmation of the flocculant property of the obtained transformants may also be accomplished by the method described in the Examples.

The non-uracil-requiring flocculant alcohol-fermenting yeast of the invention will now be explained. The non-uracil-requiring flocculant alcohol-fermenting yeast of the invention has the *Saccharomyces cerevisiae-derived* URA3 gene introduced at only one of the two LEU2 loci on the chromosomes of *Saccharomyces cerevisiae* strain FSC27. This yeast may be produced by the aforementioned production method for non-uracil-requiring flocculant alcohol-fermenting yeast of the invention.

The non-uracil-requiring flocculant alcohol-fermenting yeast of the invention exhibits a fermentation speed and alcohol production superior to yeast strain FSC27 even when cultured in uracil-free medium. Consequently, the non-uracil-requiring flocculant alcohol-fermenting yeast of the invention can be utilized for industrial alcohol production.

*Saccharomyces cerevisiae* strain FSCU-L18 has notably superior alcohol-producing ability among non-uracil-requiring flocculant alcohol-fermenting yeasts of the invention, and is therefore suitable for utilization in industrial alcohol production.

### EXAMPLES

The present invention will now be explained in greater detail through examples, with the understanding that the invention is not limited to the examples.

### (Experiment methods)

### (1) Plasmids

Two different plasmids, YIp5 and YRpGL10, were used. YIp5 is a general yeast-*E. coli* shuttle vector which has the *Saccharomyces cerevisiae* URA3 sequence as a selective marker, without the autonomous replicating sequence in yeast or centromere sequence. YRpGL10 is a plasmid made by addition of the *Saccharomyces cerevisiae* LEU2 sequence and G418 drug resistance gene sequence as selective markers to the general yeast-*E*. *coli* shuttle vector YRp7.

### (2) Yeasts

Yeast strains 396-9-6V and FSC27 were used. Strain 396-9-6V is a non-flocculant alcohol-fermenting yeast. Strain FSC27 is a strain having the flocculant gene FLO1 introduced at the URA3 locus on the strain 396-9-6V chromosome, and is a flocculant alcohol-fermenting yeast (see Japanese Patent No. 3040959).

### (3) Media

The yeast culturing was carried out using YPD medium, SD medium (glucose minimal medium) and molasses medium (16-24% sugar concentration, fermentation test medium). The compositions of each of the media were as follows. YPD medium: 10 g/L bactoyeast extract (Difco), 20 g/L bactopeptone (Difco), 10 g/L glucose, pH 5.5. YM medium: 3 g/L bactoyeast extract, 3 g/L bactomalt extract (Difco), 3 g/L bactopeptone (Difco), 10 g/L glucose, pH 4.5. SD medium (glucose minimal medium): 6.7 g/L Yeast nitrogen base without amino acids (Difco), 20 g/L glucose, pH 5.4. Molasses medium: Thai or Indonesian molasses diluted with water to 16-24% reducing sugar concentration.

After preparation, the media except for the molasses medium were subjected to autoclave treatment at 121°C for 15 minutes. Solidification of the medium was accomplished by addition of 2% agar to form a plate. For amino acid addition to the medium, an amino acid solution (sterile) of appropriate concentration was prepared and added to a concentration of 20 µg/mL after the autoclaved medium had been cooled to 60-70°C.

For plate culturing, a platinum loop was used for streaking or a spreader was used for smearing, and inversion culturing was carried out at 30°C for 2-3 days. For liquid culturing, 2 mL of medium was placed in a pilot test tube or 10 mL of medium was placed in a Monod test tube, and a sterilized bamboo skewer or toothpick was used to collect single colonies on the plate prior to seeding and shake culturing at 30°C for 1-2 days.

### (4) Yeast transformants

The yeast were transformed by the lithium acetate method. More specifically, 10-100 µg of PCR product amplified by PCR described in (6) below was introduced into strain FSC27 by the lithium acetate method for transformation. After transformation, the yeast were cultured in SD medium and strains in which the uracil requirement was restored were selected.

The lithium acetate method was carried out according to the method of H. Ito et al. (Journal of Bacteriology, Vol.153, pp.163-168(1983)). Specifically, one platinum loop of host cells was seeded from the plate to a Monod test tube containing 10 mL of YPD medium, and culturing was carried out overnight at 30°C. The culture solution was transferred to a sterilized centrifugation tube, centrifuged at 3,000 rpm for 5 minutes and suspended in TE buffer (10 mM Tris-HCl, 1 mM EDTA, pH 7.5) for washing. The washing procedure was repeated twice, and the cells collected by centrifugation were suspended in 0.25 mL of TE buffer and 0.25 mL of 0.2 M lithium acetate, the mixture was thoroughly stirred, and shaking was performed for 2 hours at 30°C. A 100 µL portion of each suspension was dispensed into a sterilized tube and gently mixed with 10 µL of DNA solution, after which the mixture was stationed at 30°C for 30 minutes. After adding 100 µL of 70% polyethylene glycol 4000 (Nacalai Tesque, Inc.) and thoroughly stirring, the mixture was stationed at 30°C for 1 hour. After further stationing at 42°C for 5 minutes and standing at room temperature, 1 mL of sterilized water was added, the mixture was stirred and centrifuged at 8,000 rpm for 1 minute. The supernatant was removed, and the precipitate was suspended in 100 µL of sterilized water and then spread onto selection plate medium. The plate was cultured for 3-5 days at 30°C.

### (5) Preparation of total yeast DNA and Southern blotting

The total yeast DNA was prepared according to the method of P. Philippsen et al. (Methods in Enzymology, Vol.194, pp.169-182(1990)). A 500 µL portion of the cell solution which had been cultured overnight was seeded in an L-shaped test tube containing 10 mL of medium. After shake culturing overnight at 30°C until the stationary phase, the medium was transferred into a centrifugation tube and centrifuged at 3,000 rpm for 5 minutes, and then the supernatant was discarded, and the precipitate was suspended in 1 mL of 1.2 M sorbitol solution and transferred to an Eppendorf tube. The solution was centrifuged at 15,000 rpm for 1 minute, the supernatant was discarded, and the precipitate was suspended in 500 µL of 1.2 M sorbitol-50 mM Tris-HCl solution (pH 7.5). To this there was then added 10 µL of β-mercaptoethanol and 50 µL of Zymolyase solution (5 mg/mL Zymolyase 20T, 50 mM Tris-HCl, pH 7.5), and the resulting mixture was stationed at 30°C for 1 hour for protoplasting. After centrifuging at 5,000 rpm for 1 minute and discarding the supernatant, 500 µL of 0.2% SDS-50 mM EDTA solution (pH 8.0) was added and mixed therewith, and the mixture was stationed at 70°C for 15 minutes. A sufficient amount of Proteinase K was then added and mixed therewith, and the mixture was stationed at 37°C for 1 hour. After adding 100 µL of 5 M potassium acetate (pH 4.8) and gently mixing, the mixture was cooled on ice for 30 minutes. It was then centrifuged at 8000 rpm for 1 minute, the supernatant was transferred to a separate tube, 500 µL of TE-saturated phenol was added to prepare a thorough suspension, centrifugation was performed at 4°C, 15,000 rpm for 10 minutes, and the upper layer (aqueous phase) was transferred to a separate tube. After then adding 500 µL of phenol-chloroform and preparing a thorough suspension, centrifugation was performed at 15,000 rpm for 5 minutes and the aqueous phase was transferred to a separate tube. Isopropanol was added to the mouth of the tube and a thorough suspension was prepared and allowed to stand for 30 minutes, after which centrifugation was performed at 15,000 rpm for 5 minutes and the supernatant was discarded. The precipitate was washed with 70% ethanol, dried and dissolved in 20 µL of TE buffer containing 50 µg/mL of RNase A. After standing at room temperature for 30 minutes, 80 µL of TE buffer and 100 µL of 5 M ammonium acetate were added and mixed therewith, and the mixture was stationed in ice for 30 minutes. Centrifugation was performed at 4°C, 15,000 rpm for 10 minutes, the supernatant was transferred to a separate tube, 1 mL of ethanol was added and the mixture was placed in dry ice for 10 minutes. Centrifugation was performed at 4°C, 15,000 rpm for 10 minutes, the supernatant was discarded, and then the precipitate was washed with 70% ethanol, dried and dissolved in 20 µL of TE buffer.

Southern blotting was carried out using an ECL direct nucleic acid labeling and detection system (Amersham). For hybridization and probe labeling, the DNA probe was treated at 100°C for 5 minutes and then rapidly cooled on ice for denaturation, reacted with the DNA-labeling reagent and glutaraldehyde at 37°C for 10 minutes, and labeled with peroxidase. A membrane was placed in Gold hybridization buffer and pre-hybridization was carried out at 42°C for at least 20 minutes. The probe was added thereto for overnight hybridization. The membrane was washed twice in washing buffer (0.5xSSC, 6 M urea, 0.4% SDS) at 42°C for 20 minutes. It was washed two more times with 2xSSC for 5 minutes, and ECL detection reagent was added and mixed therewith. The DNA on the membrane on which hybrids with the probe were formed was reacted with peroxidase and hydrogen peroxide in detection reagent, resulting in oxidation of the luminol in the detection reagent, luminescence, and detection thereof as a signal by photosensitivity on a film.

The analysis procedure for Southern blotting was carried out using a VacuGene XL Vacuum Blotting System (Pharmacia). Specifically, the electrophoresed gel was placed on a Hybond N+ (Amersham) nylon membrane and set in a blotting apparatus, and then subjected to suction blotting at 50 millibars in different solutions. The solutions used and the treatment times were as follows: (1) Acid treatment solution: 0.25 M hydrochloric acid, 7 min, (2) Denaturing solution: 0.5 M sodium hydroxide solution containing 1.5 M sodium chloride, 7 min, (3) Neutralizing solution: 1.0 M Tris buffer (pH 7.5) containing 1.5 M sodium chloride, 7 min, (4) Transfer solution: 20xSSC, 30 min. Blotting was followed by alkali fixing treatment of the membrane.

### (6) PCR

The PCR was carried out using a GeneAmp^{™} PCR System 9600 (Perkin-Elmer). The obtained PCR product was purified by agarose gel electrophoresis and then used in tests for transformation, etc. The detailed PCR conditions for each PCR product are shown below.

### (6-1) URA3 ORF

Template: PstI fragment of YIp5 (containing laboratory yeast-derived URA3)
Primers:
URA3P1: ATGTCGAAAGCTACATATAAGGA (SEQ ID NO. 1)
URA3P2: TTAGTTTTGCTGGCCGCATC (SEQ ID NO. 2)
DNA polymerase: Expand^{™} High Fidelity enzyme mix (Boehringer Mannheim)
Reaction solution composition (total of 50 µL):
10 mM PCR nucleotide mix (Boehringer Mannheim), 1 µL
Template DNA (0.1 µg/µL), 1 µL
50 µM primer URA3P1, 1 µL
50 µM primer URA3P2, 1 µL
10xPCR buffer, 5 µL
DNA polymerase, 0.75 µL
Water, 40.25 µL
Reaction conditions:
94°C, 2 min
(94°C, 15 sec; 60°C, 30 sec; and 72°C, 90 sec) x 25 cycles and
72°C, 7 min

### (6-2) LEU2 ORF

Template: BamHI fragment of YRpGL10 (containing laboratory yeast-derived LEU2)
Primers:
L2ORFP1: ATGTCTGCCCCTAAGAAGATCGT (SEQ ID NO. 3)
L2ORFP2: AAGCAAGGATTTTCTTAACTTCT (SEQ ID NO. 4)
DNA polymerase: TAKARA Ex Taq (Takara Shuzo)
Reaction solution composition (total of 50 µL):
10 mM PCR nucleotide mix, 1 µL
Template DNA (0.1 µg/µL), 1 µL
50 µM primer L2ORFP1, 1 µL
50 µM primer L2ORFP2, 1 µL
10xPCR buffer, 5 µL
DNA polymerase, 0.5 µL
Water, 40.5 µL
Reaction conditions:
94°C, 2 min
(94°C, 15 sec; 55°C, 30 sec; and 72°C, 90 sec) x 25 cycles and 72°C, 7 min

(6-3) LEU2 of strain 396-9-6V
Template: Total DNA of strain 396-9-6V
Primers:
LEU2NP1: CGCCTGACGGATATACCTTN (SEQ ID NO. 5)
LEU2NP2: GCCTACCCTATGAACATATN (SEQ ID NO. 6)
DNA polymerase: Expand^{™} High Fidelity enzyme mix
Reaction solution composition (total of 50 µL):
10 mM PCR nucleotide mix, 1 µL
Template DNA (0.1 µg/µL), 1 µL
50 µM primer LEU2NP1, 1 µL
50 µM primer LEU2NP2, 1 µL
10xPCR buffer, 5 µL
DNA polymerase, 0.75 µL
Water, 40.25 µL
Reaction conditions:
94°C, 2 min
(94°C, 15 sec; 46°C, 30 sec; and 68°C, 90 sec) x 30 cycles and
↓
72°C, 7 min

(6-4) LEU2-disrupting DNA fragment containing full-length URA3
The title DNA fragment was amplified not by recombinant DNA technology but by double fusion PCR (D.C. Amberg et al., Yeast, Vol. 11, pp. 1275-1280, 1995). The DNA polymerase for PCR used was an Expand^{™} High Fidelity enzyme mix which is resistant to errors during extension. Where necessary, the amplified intermediate PCR product was subjected to fractionation by agarose gel electrophoresis and DNA recovery from the gel, and then purification by phenol-chloroform treatment and ethanol precipitation.

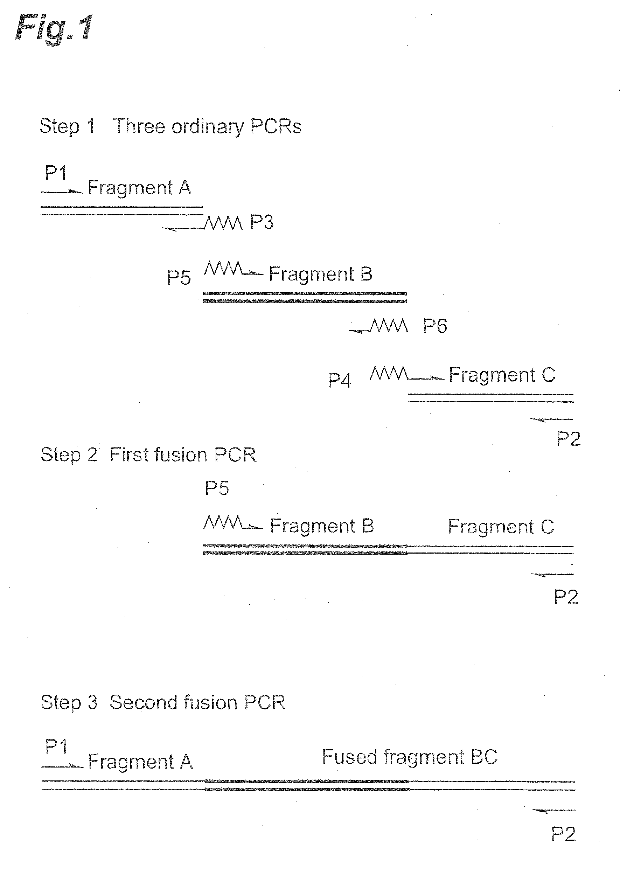

The double fusion PCR may be summarized as follows (see Fig. 1). First, ordinary PCR reaction was carried out 3 times in Step 1, first fusion PCR was carried out in Step 2, and second fusion PCR was carried out in Step 3. In Step 1, PCR was used for amplification of three different DNA fragments, a DNA fragment containing 250 bp upstream of LEU2 (the region preceding the LEU2 ORF) and the 20 bp URA3 upstream terminal for overlapping in fusion (hereinafter referred to as "fragment A"), a DNA fragment containing the full-length URA3 (hereinafter referred to as "fragment B") and a DNA fragment containing the 20 bp downstream terminal of URA3 for overlapping in fusion and 250 bp downstream of LEU2 (the region at the C-terminal end within the LEU2 ORF) (hereinafter referred to as "fragment C"). In Step 2, the fragments B and C were used as combined templates in fusion PCR, for amplification of the fused fragment BC. In Step 3, the fragment A and the fused fragment BC were used as combined templates in fusion PCR, for amplification of the fused fragment ABC.

The template DNA and primers used for each step (with the URA3-corresponding sequences underlined) were as follows.

### Step 1

Fragment A
Template: BamHI fragment of YRpGL10
Primers:
P1: TTTCAACTGAAAAATTGGGA (SEQ ID NO. 7)
P3: ATGAATTGAATTGAAAAGCTCATAGGGGCAGACATTAGAA
(SEQ ID NO. 8)
Annealing temperature: 50°C
Fragment B
Template: HindIII fragment of YIp5
Primers:
P5: AGCTTTTCAATTCAATTCAT (SEQ ID NO.9)
P6: AGCTTTTTCTTTCCAATTTT (SEQ ID NO. 10)
Annealing temperature: 46°C
Fragment C
Template: BamHI fragment of YRpGL10
Primers:
P2: TTTCAACTGAAAAATTGGGA (SEQ ID NO. 11)
P4: AAAATTGGAAAGAAAAAGCTTTTGTACGAACCATGCCACG
(SEQ ID NO. 12)
Annealing temperature: 50°C

### Step 2

Templates: DNA fragments B and C
Primers: P2 and P5
Annealing temperature: 50°C

### Step 3

Templates: DNA fragment A and fused DNA fragment BC
Primers: P1 and P2
Annealing temperature: 56°C

The reaction solution compositions for each step were as follows.

### Step 1

Reaction solution composition (total of 50 µL):
10 mM PCR nucleotide mix, 1 µL
Template DNA (0.1 µg/µL), 1 µL
50 µM forward primer, 1 µL
50 µM reverse primer, 1 µL
10xPCR buffer, 5 µL
Expand^{™} High Fidelity enzyme mix, 0.75 µL Water, 40.25 µL

### Steps 2 and 3

Reaction solution composition (total of 50 µL):
10 mM PCR nucleotide mix, 1 µL
Template DNA (mixture of 0.5 µg/µL of each fragment at equimolar concentrations), 5 µL
50 µM forward primer, 0.5 µL
50 µM reverse primer, 0.5 µL
10xPCR buffer, 5 µL
Expand^{™} High Fidelity enzyme mix, 0.75 µL Water, 37.25 µL

The following reaction conditions were the same for all of the steps, except for the annealing temperature. Reaction conditions:
94°C, 2 min
(94°C, 15 sec; annealing temperature, 30 sec; and 72°C, 90 sec) x 25 cycles and
72°C, 7 min

### (7) Yeast flocculant property stability confirmation test

The yeast cells were shake cultured overnight in 5 mL of YPD medium (2% glucose), and then 0.1 mL of medium was repeatedly subcultured in fresh medium to determine the flocculant property of the yeast. The flocculating strength was visually evaluated and judged on a 6-level scale, from the flocculant property exhibited by strain FSC27, as very strong flocculation (level 5), to non-flocculation (level 0). The culturing was carried out in two series.

### (8) Test tube fermentation test

10 mL of YPD medium (24% sugar concentration) and molasses medium were placed in an L-shaped test tube and 0.5 mL of a pre-culturing solution which had been cultured overnight were seeded thereinto, then they were shake cultured at 32°C, 80 rpm for 144 hours. Upon completion of culturing, the ethanol concentration was measured by gas chromatography.

### (9) Flask fermentation test

In 285 mL of 24% molasses medium (total sugar: 50.68%) in an Erlenmeyer flask equipped with a Mycel fermentation tube there was inoculated 15 mL of total culturing solution (YM medium, 24 hrs culturing at 32°C (48 hrs culturing of strain FSC27 alone)) (5% yeast mash), and continuous stir culturing was carried out for 6 days with a stirrer bar at 32°C. The culturing was carried out in two series.

The analysis and methods were as follows.
Alcohol concentration: Gas chromatography
Total sugar concentration: Lane method
Residual sugar concentration: Somogyi modified method
Acidity: Titration with 0.1 N aqueous solution of sodium hydroxide
pH: Measurement with pH meter
Yeast concentration: Counting with hemocytometer
Fermentation speed: Measurement (weight) of time-dependent change in carbon dioxide generation using gas meter
Impurities in test tank solution: Gas chromatography

### (10) Repeated batch fermentation test

One platinum loop of cells was seeded from slant medium into 20 mL of YM medium and shake cultured at 32°C for 24 hours (48 hours for strain FSC27 alone), as pre-preculturing. A 10 mL portion of the pre-preculture solution was seeded into 230 mL of sugar solution (16% sugar concentration) and shake cultured at 32°C for 24 hours (48 hours for strain FSC27 alone) as pre-culturing. Next, using a 3 L volume jar fermenter (2 L real volume) as the fermentation vessel, the total culture solution was seeded in molasses medium (total sugars: 50.68%, 48.41% only for Cycle #10) and main culturing was carried out at 30°C, 150 rpm.

The main culturing was carried out as follows. Cycle #1 was carried out at 0.05 VVM for 30 hours, and Cycles #2-10 were carried out at 0.05 VVM for 2 hours, with aeration.
Cycle #1: Seeding of 200 mL of pre-culturing solution in 1800 mL of sugar solution (18% sugar concentration), and culturing for 48 hours.
Cycle #2: With 20% yeast mash, addition of 1600 mL of sugar solution (18% sugar concentration) and culturing for 24 hours.
Cycle #3: With 20% yeast mash, addition of 1600 mL of sugar solution (20% sugar concentration) and culturing for 24 hours.
Cycles #4-10: With 20% yeast mash, addition of 1600 mL of sugar solution (22% sugar concentration) and culturing for 24 hours.

The following were analyzed for each cycle.
Alcohol concentration: Gas chromatography, alcoholmeter
Total sugar concentration: Lane method
Residual sugar concentration: Somogyi modified method
Acidity: Titration with 0.1 N aqueous solution of sodium hydroxide
pH: Measurement with pH meter
Total yeast count: Counting with hemocytometer
Viable cell count: Methylene blue staining
Fermentation speed: Measurement (weight) of time-dependent change in carbon dioxide generation using gas meter

### (Example 1: Introduction of URA3 into LEU2 locus of strain FSC27)

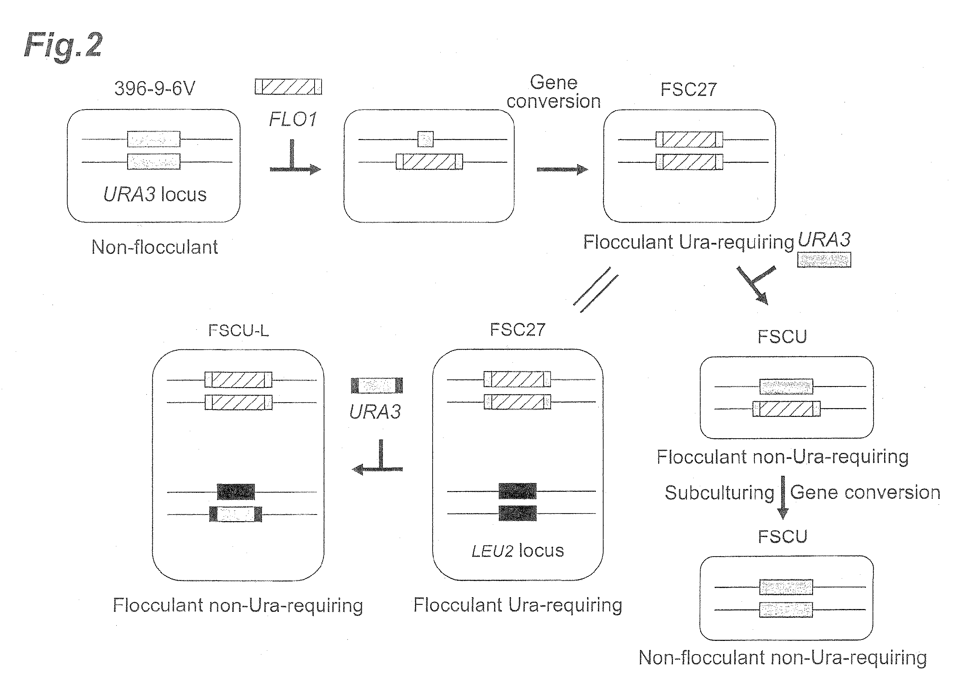

According to the examination of introducing a DNA fragment containing the full-length URA3 into a location other than the URA3 locus of strain FSC27, LEU2 was selected as the target. Since strain FSC27 is polyploid, it potentially has more than one LEU2 gene. In this example, one of the LEU2 genes was disrupted by introduction of URA3, compensating for the uracil requirement of strain FSC27. Although gene conversion was expected after gene introduction, this was thought to be prevented by maintaining the transformants in SD minimal medium. Fig. 2 shows the strategy for this example.

a. Confirmation of presence of multiple LEU2 genes in strain 396-9-6V and FSC27, and confirmation of homology with laboratory yeast-derived LEU2
   Primers (L2ORFP1 and L2ORFP2) were designed for amplification of LEU2 ORF, based on the nucleotide sequence of laboratory yeast LEU2. The primers were used for PCR with LEU2 (laboratory yeast-derived) on plasmid YRpGL10 as the template, and an approximately 1 kb DNA fragment was amplified. The PCR product was digested with different restriction endonucleases (EcoRI, EcoRV, HinfI), yielding a large fragment of the approximate expected size, and this PCR product was confirmed to be the DNA fragment containing the LEU2 ORF.

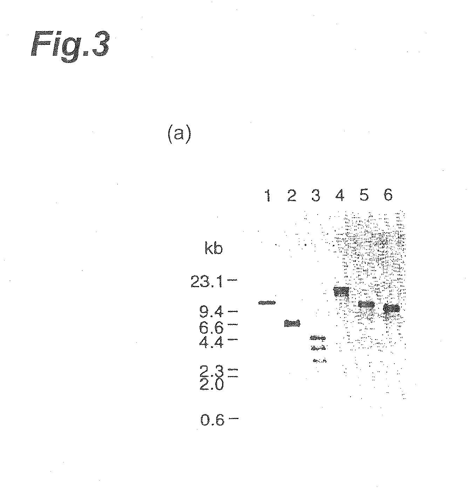

The PCR product was purified by agarose gel electrophoresis and used as a probe for Southern blotting of total DNA from strain 396-9-6V and FSC27 digested with the different restriction endonucleases. The analysis results for strain 396-9-6V are shown in Fig. 3 (since both strains showed the same pattern, only the results for strain 396-9-6V are shown). The LEU2 ORF hybridization signal was found in both strains, confirming the presence of LEU2 homologous genes in both strains. The signal patterns of both strains were also identical. Three to four signals were obtained when digestion was with restriction endonucleases EcoRI (lane 3) and EcoRV (lane 2) which have a single cleavage site within the laboratory yeast LEU2 ORF, while 1-2 signals were obtained when digestion was with a restriction endonuclease without a cleavage site in the laboratory yeast LEU2 ORF. In the case of a single copy of LEU2 in strain 396-9-6V and FSC27, digestion with EcoRI and EcoRV should yield 1-2 signals, and three or more signals should never be observed. Thus, it was conjectured that more than 2 copies of LEU2 are present in strain 396-9-6V and FSC27. This strategy assumes that LEU2 of strain FSC27 is homologous with laboratory yeast LEU2, and that at least two copies are present. Since these results confirmed the feasibility of the method, the following experiment was carried out.

b. Amplification of LEU2-disrupting DNA fragment containing full-length URA3 by PCR
   Double fusion PCR was carried out to construct a LEU2-disrupting DNA fragment containing the full-length URA3 gene. Digestion of the final PCR product with restriction endonucleases (PstI, SmaI and a mixture of both) yielded the expected cleavage pattern, confirming amplification of a LEU2-dismpting DNA fragment containing the full-length URA3.

c. Transformation of strain FSC27 and selection of obtained transformants
   The DNA fragment constructed in b. above was used in amounts of 0, 10, 50 and 100 µg to transform strain FSC27 by the lithium acetate method. The experiment was carried out twice, producing a total of 147 strains (designated as strain FSCU-L) having the uracil requirement compensated. The transformation frequency is summarized in Table 1.

**[Table 1]**

| Introduced DNA amount (µg) | Obtained transformants (colonies) | | |
|---|---|---|---|
| | 1st time | 2nd time | Total |
| 0 | 0 | 0 | 0 |
| 10 | 1 | 5 | 6 |
| 50 | 10 | 41 | 51 |
| 100 | 21 | 69 | 90 |

Upon culturing 25 arbitrarily selected strains among the 147 obtained strains in SD medium, flocculation was observed in all of the strains. The strength of flocculation was very high (level 5), equivalent to the parent strain FSC27.

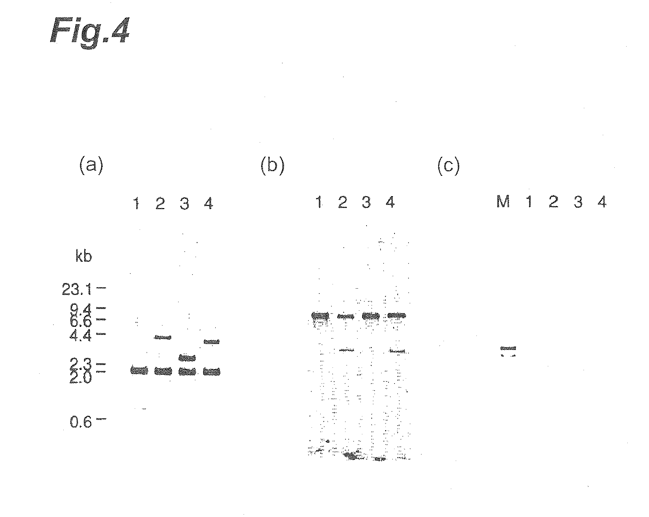

d. Confirmation of transformants
   Three of the 25 FSCU-L strains confirmed to be flocculant were subjected to Southern blotting using the LEU2 ORF as a probe. The results of the Southern blotting are shown in Fig. 4. The strength of the signal of the same size as the signal observed for strain FSC27 and 396-9-6V was attenuated, and a signal shifted toward a different size was detected. This indicated that the target gene had been introduced at one of the multiple LEU2 loci. Also, Southern blotting using URA3 ORF as a probe resulted in detection of a signal in strain FSCU-L which was not found in strain FSC27. That is, it indicated introduction of the exogenous URA3. Upon Southern blotting using pBR322 as a probe, no signal was detected in strain FSCU-L, similar to strain FSC27 and 396-9-6V. In other words, it was demonstrated that these strains are self-cloning strains without introduction of *E. coli*-derived DNA. The above results confirmed that the target gene had been incorporated into strain FSCU-L, and that the incorporation was self-cloning.

e. Confirmation of flocculant property maintenance in transformants
   Three of the 25 FSCU-L strains confirmed to be flocculant were arbitrarily selected and cultured in SD medium and YPD medium, and repeatedly subcultured 20 times, after which the flocculant property of the yeast before and after subculturing was examined. The results are shown in Table 2.

**[Table 2]**

| Strain | Flocculant property | | | |
|---|---|---|---|---|
| | SD medium | | YPD medium | |
| | Before subculturing | After subculturing | Before subculturing | After subculturing |
| FSC27 | 5 | 5 | 5 | 5 |
| FSCU-L1 | 5 | 5 | 5 | 5 |
| FSCU-L2 | 5 | 5 | 5 | 5 |
| FSCU-L3 | 5 | 5 | 5 | 5 |

The flocculant property of the yeast was maintained in all of the media, even after 20 subculturings.

### (Test Example 1: strain FSCU-L fermentation performance evaluation test)

YPD medium with a 24% sugar concentration was used for a test tube fermentation test with 25 FSCU-L strains which were confirmed to be flocculant, and 10 strains with high product alcohol concentrations were selected. Also, molasses medium with a 24% sugar concentration was used for a test tube fermentation test using these 10 strains, and the product alcohol concentrations were measured. Finally, 3 strains which had overall high product alcohol concentrations (strain FSCU-L18, 20 and 21) were selected from the media.

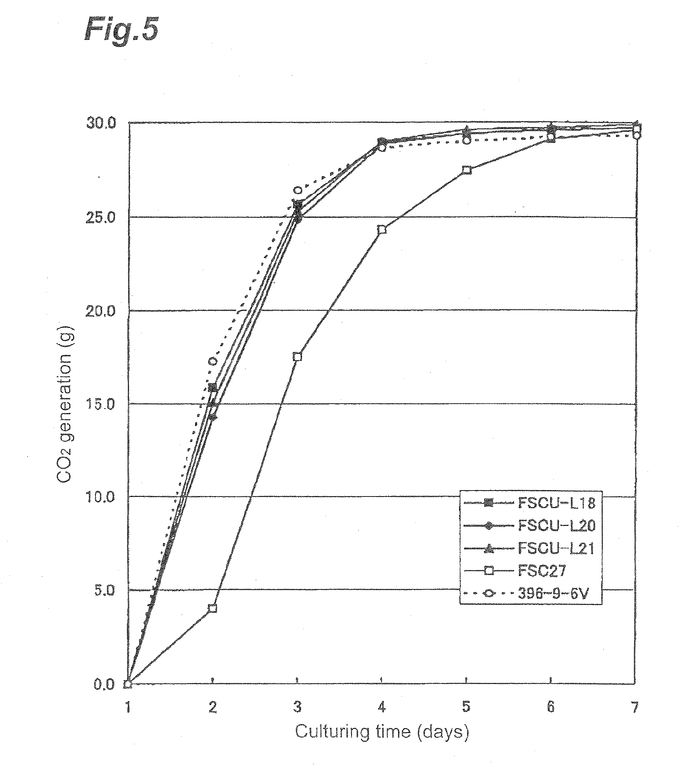

These 3 strains were subjected to a flask fermentation test using molasses medium, and the fermentation performance was evaluated in detail. The time-dependent changes in carbon dioxide generation are shown in Fig. 5, the fermented mash analysis results are shown in Table 3, and the test tank solution impurity analysis results are shown in Table 4.

**[Table 3]**

| Strain | Alcohol conc. (vol%) | Residual sugar (wt%) | Yeast count (x 10⁸/mL) | Acidity | pH | Fermentation rate (%) |
|---|---|---|---|---|---|---|
| FSCU-L18 | 12.33 | 2.41 | 1.27 | 9.23 | 5.07 | 82.62 |
| FSCU-L20 | 12.21 | 2.41 | 1.37 | 9.37 | 5.09 | 81.81 |
| FSCU-L21 | 12.23 | 2.42 | 1.14 | 9.15 | 5.09 | 81.96 |
| FSC27 | 12.13 | 2.47 | 0.79 | 10.54 | 4.99 | 81.29 |
| 396-9-6V | 12.08 | 2.79 | 1.46 | 9.23 | 5.05 | 80.93 |

**[Table 4]**

| Impurity | FSCU-L18 | FSCU-L20 | FSCU-L21 | FSC27 | 396-9-6V |
|---|---|---|---|---|---|
| Methanol | 7.7 | 7.3 | 8.1 | 7.8 | 7.2 |
| Acetaldehyde | 53.8 | 29.0 | 20.7 | 41.5 | 32.5 |
| Acetone | 1.5 | 1.3 | 1.7 | 1.2 | 1.7 |
| i-Propanol | - | - | - | - | - |
| Methyl acetate | - | - | - | - | - |
| n-Propanol | 54.9 | 57.9 | 54.9 | 54.1 | 35.0 |
| Diacetyl | 13.4 | 12.2 | 12.7 | 11.9 | 10.9 |
| Acetic acid | 481.5 | 450.1 | 511.7 | 485.9 | 417.3 |
| Ethyl acetate | 10.2 | 12.8 | 13.1 | 10.5 | 6.2 |
| i-Butanol | 37.2 | 38.7 | 37.0 | 67.0 | 41.5 |
| n-Butanol | 1.4 | 1.6 | 1.8 | 1.3 | 1.3 |
| Acetoin | 31.2 | 33.0 | 23.7 | 35.2 | 20.8 |
| i-Amyl alcohol | 126.1 | 170.3 | 161.8 | 147.7 | 174.2 |
| 2,3-Butanediol | 210.6 | 221.9 | 233.9 | 201.9 | 118.8 |
| Total impurities | 1029.5 | 1035.2 | 1081.1 | 1066.0 | 867.4 |
| Ethanol | 12.33 | 12.21 | 12.23 | 12.13 | 12.08 |

| | | | | | |
|---|---|---|---|---|---|
| Units: ppm (vol% for ethanol), -: below detection limit | | | | | |

As shown in Fig. 5, the fermentation speeds of the three FSCU-L strains were roughly equivalent to strain 396-9-6V, and the reduction in fermentation speed of strain FSC27 due to the requirement for uracil was improved in strain FSCU-L, the modified strain of FSC27. Also, Table 3 shows that the yeast count was improved with strain FSCU-L. The product ethanol concentration was higher with strain FSCU-L than with strain FSC27 or 396-9-6V, with strain FSCU-L18 being the highest. The flocculant property in molasses was also stronger with all of the three FSCU-L strains. Moreover, Table 4 shows for the test tank solution impurity that the composition was approximately the same for all of the strains. These results confirmed that the fermentation performance of strain FSCU-L is slightly superior to that of strain 396-9-6V.

### (Test Example 2: Examination of strain FSCU-L feasibility)

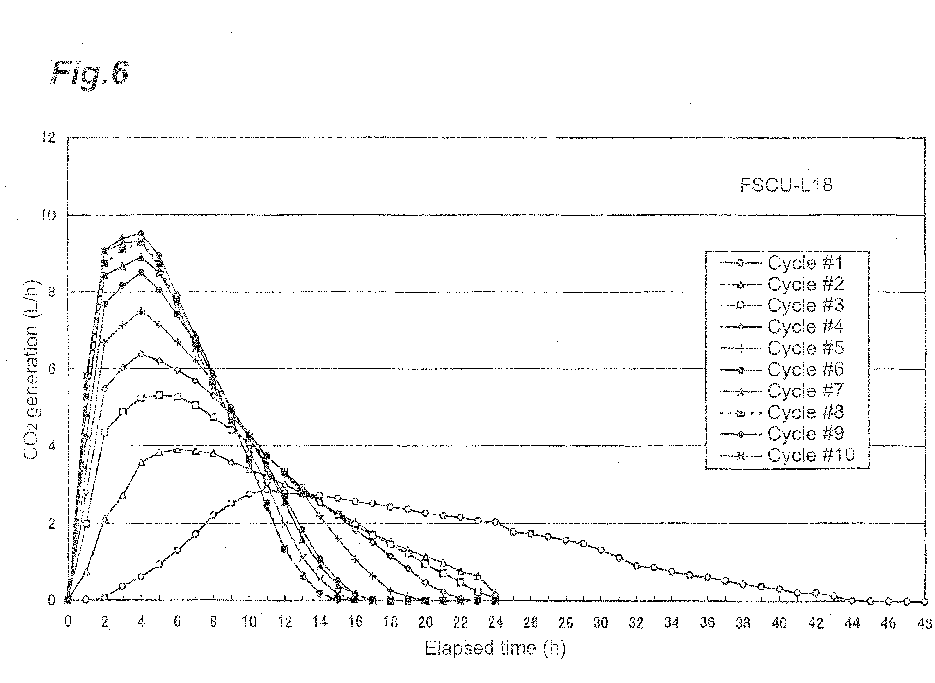

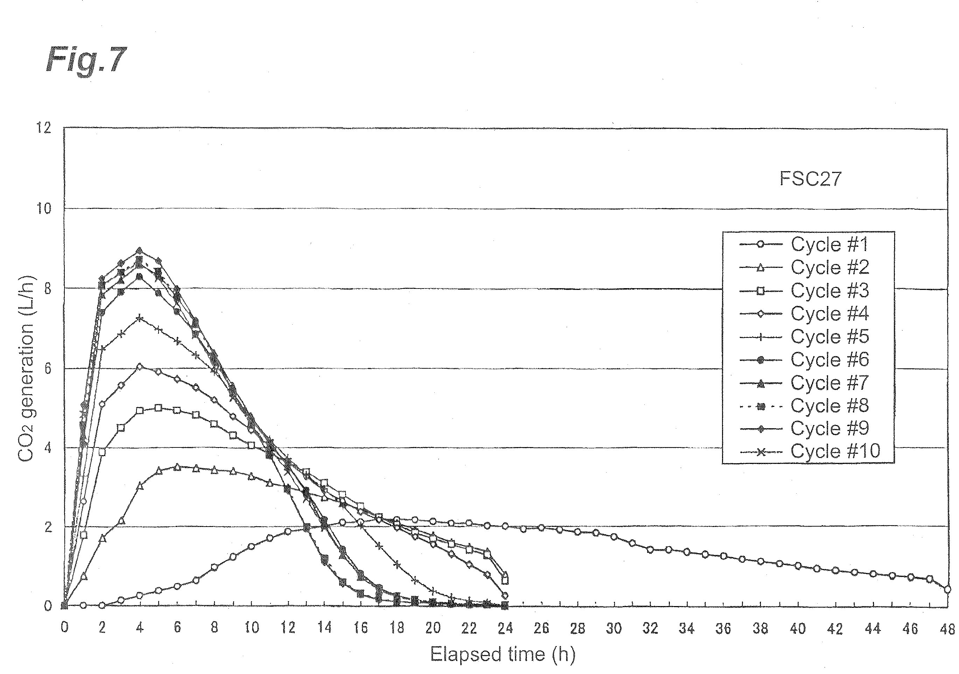

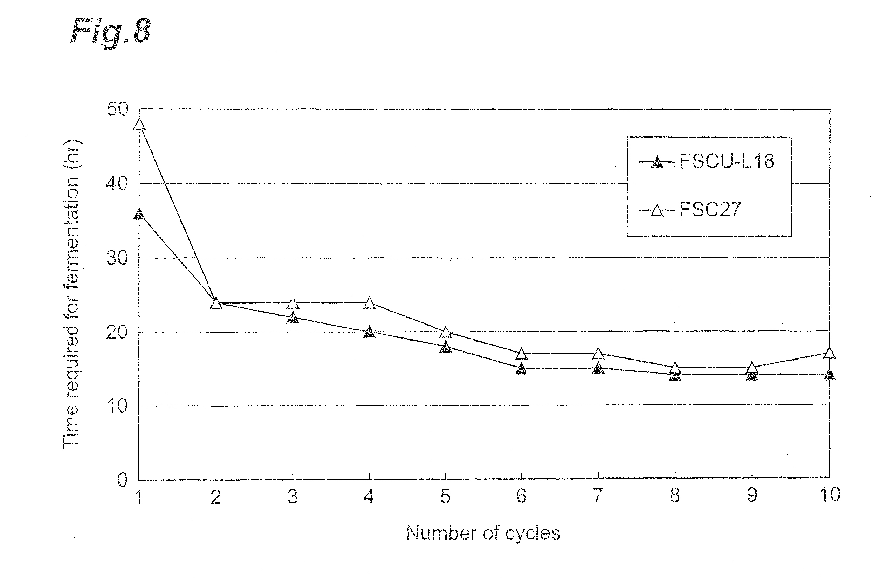

In order to examine the feasibility of strain FSCU-L, a repeated batch fermentation test was carried out with ajar fermenter (using strain FSC27 as the control). The carbon dioxide generation is shown in Figs. 6 and 7, while the fermented mash analysis results are shown in Table 5 and the time required for fermentation is shown in Fig. 8.

**[Table 5]**

| Cycle No. | | | 1 | 2 | 3 | 4 | 5 | 6 | 7 | 8 | 9 | 10 | Average |
|---|---|---|---|---|---|---|---|---|---|---|---|---|---|
| | | 24h | 48h | | | | | | | | | | |
| | TS | | 18.31 | 18.09 | 20.26 | 21.94 | 21.73 | 21.69 | 22.03 | 21.88 | 21.89 | 22.62 | 21.04 |
| | pH | | 5.21 | 5.22 | 5.19 | 5.18 | 5.18 | 5.17 | 5.15 | 5.17 | 5.17 | 5.13 | 5.18 |
| | AV | | 3.93 | 3.84 | 4.39 | 4.72 | 4.63 | 4.88 | 4.96 | 4.79 | 4.79 | 5.57 | 4.65 |
| | ALC(GC) | 6.97 | 9.82 | 9.53 | 10.83 | 11.67 | 11.39 | 11.43 | 11.53 | 11.12 | 11.29 | 11.78 | 11.04 |
| | ALC(test) | | 9.35 | 9.20 | 10.10 | 11.10 | 11.05 | 10.95 | 11.05 | 10.85 | 10.90 | 11.20 | 10.58 |
| | RTS | | 2.06 | 2.20 | 2.10 | 2.36 | 2.24 | 2.19 | 2.16 | 2.05 | 2.12 | 2.42 | 2.19 |
| FSCU-L18 | TY | 1.77 | 1.37 | 2.03 | 2.45 | 3.42 | 3.68 | 3.47 | 3.88 | 3.97 | 4.34 | 4.34 | 3.30 |
| | LCR | | 100.00 | 100.00 | 100.00 | 100.00 | 100.00 | 98.90 | 98.30 | 98.30 | 98.80 | 98.30 | 99.26 |
| | pH | | 5.01 | 5.05 | 5.04 | 5.10 | 5.11 | 5.10 | 5.10 | 5.10 | 5.10 | 5.09 | 5.08 |
| | AV | | 7.11 | 7.10 | 7.27 | 7.44 | 7.65 | 7.64 | 8.09 | 8.39 | 8.10 | 9.49 | 7.83 |
| | SR | | 88.61 | 85.22 | 87.39 | 86.87 | 87.46 | 87.70 | 88.04 | 88.57 | 88.17 | 86.93 | 87.49 |
| | FR(GC) | | 84.36 | 81.62 | 84.83 | 83.89 | 81.25 | 81.81 | 81,53 | 78.82 | 80.11 | 81.40 | 81.96 |
| | FR(test) | | 80.32 | 78.79 | 79.12 | 79.79 | 78.82 | 78.37 | 78.14 | 76.91 | 77.34 | 77.40 | 78.50 |
| | TS | | 18.31 | 18.09 | 20.26 | 21.94 | 21.73 | 21.69 | 22.03 | 21.88 | 21.89 | 22.62 | 21.04 |
| | pH | | 5.21 | 5.22 | 5.19 | 5.18 | 5.18 | 5.17 | 5.15 | 5.17 | 5.17 | 5.13 | 5.18 |
| | AV | | 3.93 | 3.84 | 4.39 | 4.72 | 4.63 | 4.88 | 4.96 | 4.79 | 4.79 | 5.57 | 4.65 |
| | ALC(GC) | 5.37 | 8.87 | 8.79 | 10.31 | 11.47 | 11.58 | 11.47 | 11.29 | 11.26 | 11.49 | 11.61 | 10.81 |
| | ALC(test) | | 8.40 | 8.45 | 9.70 | 10.90 | 11.05 | 11.00 | 11.05 | 10.80 | 10.90 | 11.10 | 10.34 |
| | RTS | | 3.35 | 3.23 | 2.64 | 2.54 | 2.30 | 2.23 | 2.18 | 2.11 | 2.17 | 2.46 | 2.52 |
| FSC27 | TY | 1.65 | 1.52 | 1.92 | 2.53 | 3.02 | 3.58 | 3.96 | 4.30 | 4.55 | 4.41 | 4.60 | 3.44 |
| | LCR | | 100.00 | 100.00 | 100.00 | 100.00 | 100.00 | 97.70 | 97.60 | 97.60 | 97.20 | 97.30 | 98.74 |
| | pH | | 4.95 | 5.01 | 5.00 | 5.07 | 5.09 | 5.07 | 5.06 | 5.06 | 5.05 | 5.04 | 5.04 |
| | AV | | 7.79 | 7.52 | 8.11 | 8.29 | 7.96 | 8.12 | 8.75 | 8.64 | 8.69 | 10.04 | 8.39 |
| | SR | | 81.47 | 78.67 | 84.34 | 85.95 | 87.15 | 87.48 | 87.94 | 88.24 | 87.90 | 86.72 | 85.59 |
| | FR(GC) | | 76.20 | 75.28 | 80.76 | 82.45 | 82.60 | 81.60 | 79.84 | 79.81 | 81.53 | 80.23 | 80.03 |
| | FR(test) | | 72.16 | 72.37 | 75.98 | 78.36 | 78.82 | 78.73 | 78.14 | 76.55 | 77.34 | 76.71 | 76.52 |

| | | | | | | | | | | | | | |
|---|---|---|---|---|---|---|---|---|---|---|---|---|---|
| TS: Total sugar (%), ALC(GC): Ethanol concentration (vol%) according to GC analysis, ALC(test): Ethanol concentration (vol%) of test tank solution according to alcoholmeter analysis, RTS: Residual total sugar (%), TY: Total yeast (x 10E+8/mL), LCR: Live cell ratio (%), AV: Acid value, SR: Sugar consumption rate (%), FR(GC): Fermentation rate (%) calculated from GC analysis value, FR(test): Fermentation rate (%) calculated from alcoholmeter analysis value. | | | | | | | | | | | | | |

The product ethanol concentration, fermentation rate and fermentation speed for strain FSCU-L18 were all superior to the parent strain FSC27, and therefore a compensating effect on uracil requirement was found. The yeast counts and viable cell rate of strain FSCU-L18 were high and the flocculant property was strong (Size of floc: 0.5-2 mm diameter; State of floc: firm, solid particles formed).

### Industrial Applicability

The present invention can provide non-uracil-requiring flocculant alcohol-producing yeast having an excellent alcohol production ability and fermentation speed.

| | | |
|---|---|---|
| 0-1 | FORM PCT/RO/134 (SAFE) | |
| 0-1-1 | The indications relating to the deposited microorganism or other biological material (PCT Rule 13bis) were prepared according to the following: | JPO-PAS 0324 |
| 0-2 | International application No. | |
| 0-3 | Applicant's or agent's file reference | FP05-0267-00 |
| | | |
| 1 | The indications made below relate to the deposited microorganism or other biological material referred to in the description | |
| 1-1 | in paragraph | 0003 |
| 1-3 | IDENTIFICATION OF DEPOSIT | |
| 1-3-1 | Name of depositary institution | IPOD: National Institute of Advanced Industrial Science and Technology, International Patent Organism Depositary |
| 1-3-2 | Address of depositary institution | Tsukuba Central 6, 1-1-1 Higashi, Tsukuba, Ibaraki, Japan, 305-8566 |
| 1-3-3 | Date of deposit | January 7, 1997 |
| 1-3-4 | Accession Number | IPOD FERM P-16023 |
| 1-5 | DESIGNATED STATES FOR WHICH INDICATIONS ARE MADE | All designated States |
| 2 | The indications made below relate to the deposited microorganism or other biological material referred to in the description | |
| 2-1 | in paragraph | 0008 |
| 2-3 | IDENTIFICATION OF DEPOSIT | |
| 2-3-1 | Name of depositary institution | IPOD: National Institute of Advanced Industrial Science and Technology, International Patent Organism Depositary |
| 2-3-2 | Address of depositary institution | Tsukuba Central 6, 1-1-1 Higashi, Tsukuba, lbaraki, Japan, 305-8566 |
| 2-3-3 | Date of deposit | May 20, 2004 |
| 2-3-4 | Accession Number | IPOD FERM P-20055 |
| 2-5 | DESIGNATED STATES FOR WHICH INDICATIONS ARE MADE | All designated States |

| For receiving Office use only | | |
|---|---|---|
| 0-4 | This sheet was received with the international application | |
| 0-4-1 | Authorized officer | |

| For International Bureau use only | | |
|---|---|---|
| 0-5 | This sheet was received by the International Bureau on: | |
| 0-5-1 | Authorized officer | |

## Claims

1. A method for production of non-uracil-requiring flocculant alcohol-fermenting yeast, comprising a step of introducing the *Saccharomyces cerevisiae-*derived URA3 gene only at one of the two LEU2 loci on the chromosomes of *Saccharomyces cerevisiae* strain FSC27 (FERM P-16023).

2. Non-uracil-requiring flocculant alcohol-fermenting yeast having the *Saccharomyces cerevisiae-*derived URA3 gene introduced only at one of the two LEU2 loci on the chromosomes of *Saccharomyces cerevisiae* strain FSC27 (FERM P-16023).

3. Flocculant alcohol-fermenting yeast according to claim 2, **characterized in that** the flocculant alcohol-fermenting yeast is *Saccharomyces cerevisiae* strain FSCU-L18 (FERM P-20055).
